(19) Europäisches Patentamt
European Patent Office
Office européen des brevets

(11) Veröffentlichungsnummer: **0 010 213**
**B1**

# (12) EUROPÄISCHE PATENTSCHRIFT

(45) Veröffentlichungstag der Patentschrift: **04.11.81**

(51) Int. Cl.³: **C 07 C 47/225,** C 07 C 49/21, C 07 C 33/05 // C11B9/00

(21) Anmeldenummer: **79103726.0**

(22) Anmeldetag: **01.10.79**

(54) **Bicyclische Verbindungen, Verfahren zu deren Herstellung und deren Verwendung.**

(30) Priorität: **07.10.78 DE 2843838**

(43) Veröffentlichungstag der Anmeldung: **30.04.80 Patentblatt 80/9**

(45) Bekanntmachung des Hinweises auf die Patenterteilung: **04.11.81 Patentblatt 81/44**

(84) Benannte Vertragsstaaten: **CH DE FR GB NL**

(56) Entgegenhaltungen:
**DE-A-2 200 090**
**DE-A-2 444 003**
**US-A-3 662 008**
**US-A-3 673 261**
**US-A-4 128 509**

(73) Patentinhaber: **BASF Aktiengesellschaft, Carl-Bosch-Strasse 38, D-6700 Ludwigshafen (DE)**

(72) Erfinder: **Baumann, Manfred, Dr. Dipl.-Chem., Im Sennteich 26, D-6800 Mannheim 24 (DE)**
Erfinder: **Hoffmann, Werner, Dr. Dipl.-Chem., Ringstrasse 11C, D-6701 Neuhofen (DE)**

**EP 0 010 213 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Bicyclische Verbindungen, Verfahren zu deren Herstellung und deren Verwendung

Die vorliegende Erfindung betrifft bicyclische Verbindungen der allgemeinen Formel I

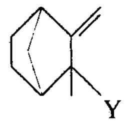
(I)

in der Y für

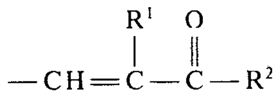
(Ia)

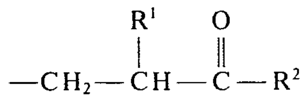
(Ib)

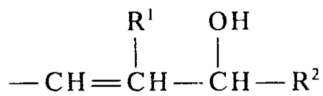
(Ic)

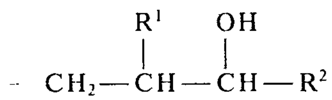
(Id)

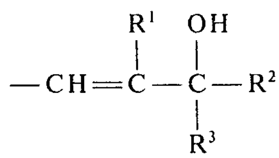
(Ie)

oder

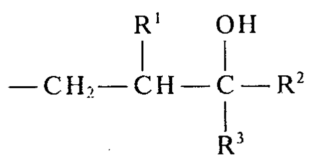
(If)

steht,
worin

$R^1$ Wasserstoff oder die Methylgruppe,
$R^2$ Wasserstoff oder eine Methyl- oder Äthylgruppe und
$R^3$ eine Methyl- oder Vinylgruppe bedeuten, ausgenommen die Verbindungen der allgemeinen Formel Ib, in der $R^1 = R^2$ = Wasserstoff sind.

Ferner betrifft die Erfindung Verfahren zur Herstellung der Verbindungen (I) sowie Synthesen mit den Verbindungen (I), insbesondere die Verwendung der Verbindungen Ia bzw. Ib zur Herstellung von bicyclischen Verbindungen der allgemeinen Formel V

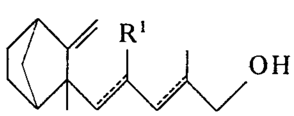
(V)

vor allem von $\beta$-Santalol.

Der Erfindung lag die Aufgabe zugrunde, den im Sandelholzextrakt enthaltenen wichtigen Duftstoff $\beta$-Santalol Va

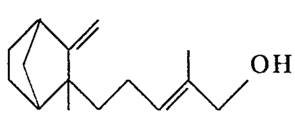
(Va)

sowie chemisch verwandte Verbindungen von Va synthetisch herzustellen, da das $\beta$-Santalol aus natürlichen Vorkommen immer knapper und dadurch teurer wird.

Mit den Verbindungen der Formeln Ia und Ib wurden Verbindungen gefunden, die einerseits auf relativ einfache Weise hergestellt werden können und die andererseits vorteilhafte Wege zur synthetischen Herstellung von $\beta$-Santalol, Dehydro-$\beta$-Santalol und anderen ähnlich gut duftenden Verbindungen eröffnen. Die übrigen neuen Verbindungen sind wertvolle Duftstoffe mit verschiedenen Holznoten.

Es wurde gefunden, daß man die Verbindungen (I) erhält, wenn man den Aldehyd II

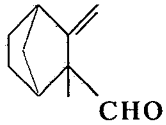

(II)

in an sich bekannter Weise nach den Methoden der gemischten Aldolkondensation mit einer Carbonylverbindung III

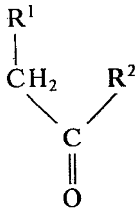

(III)

in der

$R^1$ und $R^2$ die oben angegebene Bedeutung haben, umsetzt und die hierbei entstehende Verbindung Ia gewünschtenfalls in an sich bekannter Weise zu den Verbindungen der Formeln Ib, Ic bzw. Id hydriert oder zur Herstellung der Verbindungen Ie und If die gebildete Verbindung Ia bzw. Ib in an sich bekannter Weise unter den Bedingungen von Grignard-Reaktionen mit einer Grignardverbindung der allgemeinen Formel VI

$$R^3\text{—Mg—Hal} \qquad \text{(VI)}$$

in der

$R^3$ eine Methyl- oder Vinylgruppe bedeutet und Hal für Chlor oder Brom steht, umsetzt.

Die Ausgangsverbindung II (2-Methylen-3-methyl-3-formyl-bicyclo-[2,2,1]-heptan) ist bisher weder synthetisiert noch charakterisiert worden. In Helv. Chim. Acta 59 (1976), S. 738, heißt es, daß einige experimentelle Anzeichen vermuten lassen, daß das bicyclische Teresantalal II auch im Sandelholzöl vorkommt. Der Aldehyd II kann aus dem neuen, jedoch in der nicht vorveröffentlichten Patentanmeldung P 2 719 976 beschriebenen 2-Chlormethyl-3-methyl-3-formyl-bicyclo-[2,2,1]-heptan-(5) durch HCl-Abspaltung unter Schutz der Formylgruppe durch Acetalisieren erhalten werden. Er hat einen Siedepunkt von 74 bis 78°C bei einem Druck von 0,2 Torr.

Die Umsetzung der Ausgangsverbindung II mit den Carbonylverbindungen III erfolgt in an sich bekannter Weise nach den Methoden der gemischten Aldolkondensation mit Hilfe alkalischer Kondensationsmittel. Handelt es sich bei den Carbonylverbindungen III um Aldehyde, so wird zweckmäßigerweise II vorgelegt und der Aldehyd nach Maßgabe fortschreitender Reaktion zugegeben, um auf diese Weise der Selbstkondensation der Aldehyde entgegenzuwirken. Im Falle von Ketonen als Reaktionspartner III ist eine solche Vorsichtsmaßnahme im allgemeinen nicht erforderlich, da hierbei die gemischte Aldolkondensation in der Regel wesentlich schneller verläuft, als die Selbstkondensation der Ketone.

Man nimmt die Aldolkondensation von II mit den Carbonylverbindungen III zweckmäßigerweise bei Temperaturen zwischen 40° und 180°C sowie in Gegenwart eines organischen Lösungsmittels vor.

Als Lösungsmittel kommen insbesondere destillativ leicht abtrennbare Verbindungen, z. B. Alkohole wie Methanol und Äthanol, Äther wie Dioxan und Tetrahydrofuran oder Alkohol-Wasser-Gemische in Betracht oder auch — falls III ein Keton ist — ein Überschuß des betreffenden Ketons. Die Menge der Lösungsmittel beträgt vorzugsweise das 1- bis 10fache der Menge Ausgangsverbindung II.

Als alkalisches Kondensationsmittel empfiehlt sich vor allem Kaliumhydroxid in Form einer wäßrigen oder methanolischen Lösung. Jedoch eignen sich auch andere mineralische Basen wie Natriumhydroxid und Calciumhydroxid oder starke organische Basen wie Triäthylamin, Triäthanolamin oder ein Piperidin-Eisessig-Gemisch. Die Menge der Katalysatoren beträgt — wie allgemein für Aldolkondensationen üblich — zweckmäßigerweise 0,01 bis ca. 0,3 Mol-%, bezogen auf II.

Nach erfolgter Aldolkondensation neutralisiert man das Reaktionsgemisch wie üblich mit einer beliebigen Säure, z. B. mit Essigsäure, trennt das hierbei anfallende Salz ab und arbeitet das Gemisch, ebenfalls wie üblich, auf das Aldolisierungsprodukt auf.

Der bei der Aldolkondensation zunächst entstehende Alkohol Ia'

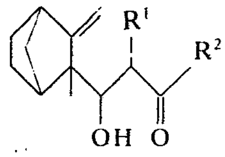

(Ia')

geht unter den Reaktionsbedingungen – besonders wenn man bei Siedetemperatur arbeitet – unmittelbar in die entsprechende olefinische Verbindung Ia über.

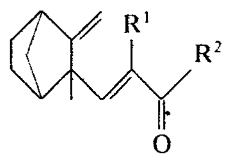

(Ia)

Die neuen Verbindungen der Formel Ia weisen einen holzigen Geruch auf. Der Geruch ist jedoch etwas synthetisch streng, so daß die Verbindungen Ia selber nur in geringeren Mengen zur Nuancierung von Duftstoffkompositionen oder für Spezialzwecke, beispielsweise zur geruchlichen Verbesserung von Chemieprodukter wie Detergentien oder Kunststoffdispersionen verwendet werden.

Das gleiche gilt für die Carbonylverbindungen Ib

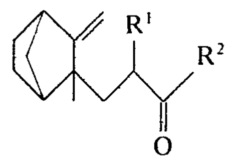

(Ib)

die man durch partielle Hydrierung von Ia in an sich bekannter Weise mit $H_2$ und Raney-Nickel oder mit desaktivierten Palladium/Kohle-Katalysatoren erhält. Die sehr stabile 2-Methylengruppe des bicyclischen Restes wird hierbei nicht angegriffen.

Hydriert man die Verbindungen Ia, ebenfalls in an sich bekannter Weise, bei 0° bis 150°C mit Reduktionsmitteln wie $NaBH_4$, $LiAlH_4$ oder Al-triisopropylat (nach Meerwein-Ponndorf), so erhält man die primären ($R^2 = H$) oder sekundären ($R^2 = -CH_3$; $-C_2H_5$) Alkohole Ic

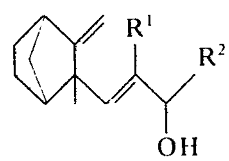

(Ic)

welche einen sehr angenehmen Geruch nach Edelholz haben und sich daher als Komponenten für kosmetische Duftstoffkompositionen eignen.

Im Vergleich dazu haben die ebenfalls recht wertvollen Duftstoffe Id

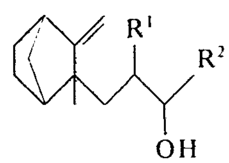

(Id)

die man in an sich bekannter Weise durch Hydrierung der Verbindungen Ib, z. B. nach Meerwein-Ponndorf, erhält, einen etwas matteren Geruch.

Der Aldehyd Ia mit $R^1 = R^2 = H$ läßt sich auch mit guten Ausbeuten herstellen, wenn man den Aldehyd II in an sich bekannter Weise mit Vinyl-Mg-halogeniden umsetzt, den erhaltenen Allylalkohol (2-Methylen-3-methyl-3(1-hydroxy-2-propen-1-yl)-bicyclo[2,2,1]-heptan) mit Phosgen oder Thionylchlorid in das 2-Methylen-3-methyl-3-(3-chlor-1-propen-1-yl)-bicyclo[2,2,1]-heptan umlagert und dieses mit Hexamethylentetramin oder den Na-Salzen von Nitroverbindungen oxidiert, wobei diese Schritte in an sich bekannter Weise erfolgen:

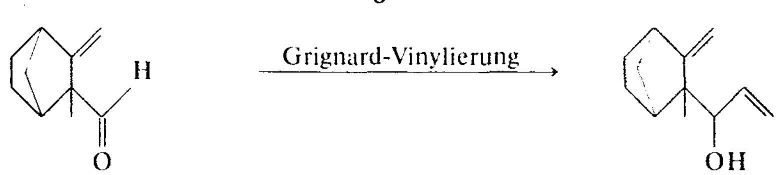

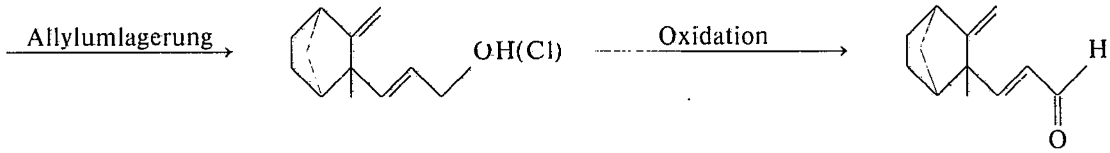


Während die Alkohole Ic und Id unmittelbar als Duftstoffe verwendet werden können, haben die Carbonylverbindungen Ia und Ib größere Bedeutung als Ausgangsstoffe für die Herstellung von β-Santalol (Va) und damit verwandter Verbindungen.

Setzt man die unter die Formeln Ia und Ib fallenden Aldehyde unter den Bedingungen der gemischten Aldolkondensation, wie sie für die Umsetzung von II mit den Carbonylverbindungen III beschrieben wurde, mit Propionaldehyd um,

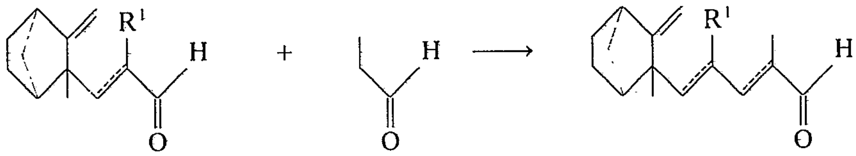

(IV)

so erhält man – gegebenenfalls nach anschließender Hydrierung der bei der Aldolkondensation neu entstehenden Doppelbindung – die Aldehyde der Formel IV. Die Bindungen ------- bedeuten hierbei eine Doppelbindung oder eine aus der Doppelbindung durch Hydrierung hervorgegangene Einfachbindung.

Durch Hydrierung der Verbindungen IV analog der Hydrierung von den Verbindungen Ia gelangt man zu den Alkoholen V

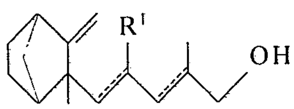

(V)

unter denen das β-Santalol Va und das Dehydro-β-Santalol Vb

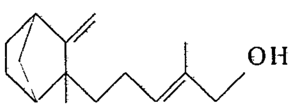

(Va)

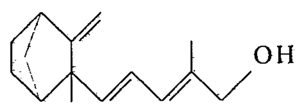

(Vb)

für die Parfümerie von größter Wichtigkeit sind.

Ein anderer vorteilhafter Syntheseweg für die Herstellung von Va und Vb wurde durch das Auffinden der Ketone Ia bzw. Ib, in denen $R^1 = H$ und $R^2$ für Äthyl steht, möglich. Hierbei werden die Ketone Ia bzw. Ib in an sich bekannter Weise (Claisen-Wislicenus-Kondensation) zunächst in Gegenwart von niederen Alkalialkanolaten mit Methylformiat und dann in Gegenwart eines sauren Katalysators mit Methanol nach folgender Reaktionsgleichung umgesetzt.

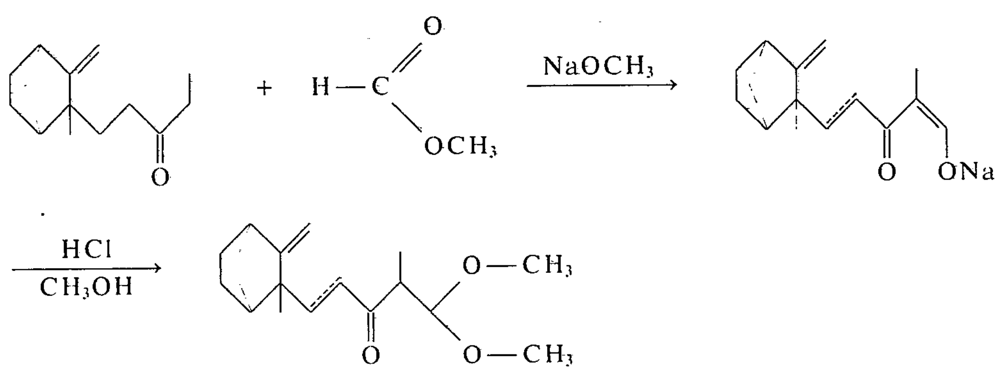


Anschließende Hydrierung der Carbonylgruppe mit Reduktionsmitteln wie $NaBH_4$, $LiAlH_4$ oder Al-tri-isopropylat (nach Meerwein-Ponndorf), Dehydratisierung, Desacetalisierung und Hydrierung der Formylgruppe zur Hydroxylgruppe ergibt Va bzw. Vb nach folgender Reaktionsgleichung:

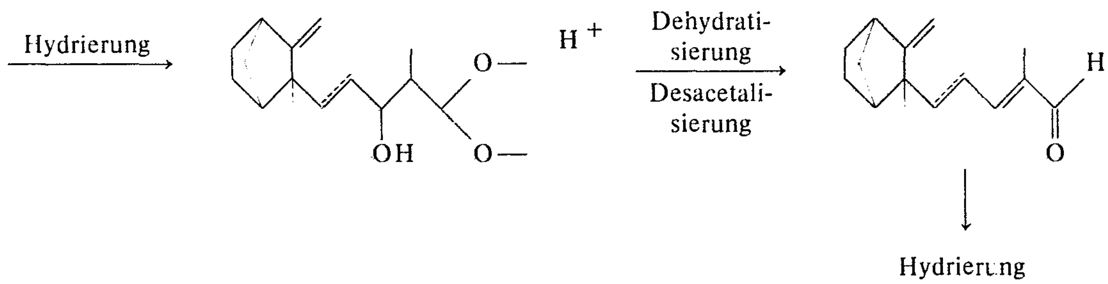


(Va und Vb)

Die erstbeschriebene Synthese von Va und Vb über eine gemischte Aldolkondensation mit Propionaldehyd bietet den Vorteil, daß sie nur aus wenigen Schritten besteht. Wegen der Selbstkondensation des Propionaldehyds erhält man jedoch ein Reaktionsgemisch, aus dem sich die Verbindungen Va und Vb mit Duftstoffqualität für höhere Ansprüche nur durch aufwendige fraktionierte Destillation reinigen läßt. Zur Herstellung der reinen Verbindungen ist daher der Weg über das Methylformiat, auch wenn er über mehrere Stufen erfolgt, günstiger.

Setzt man die Carbonylverbindungen Ia und Ib in an sich bekannter Weise mit Grignardverbindungen $R^3$—Mg—Hal (VI) um, in denen $R^3$ eine Methyl- oder Vinylgruppe und Hal, Chlor oder Brom bedeutet, so erhält man die Alkohole Ie bzw. If

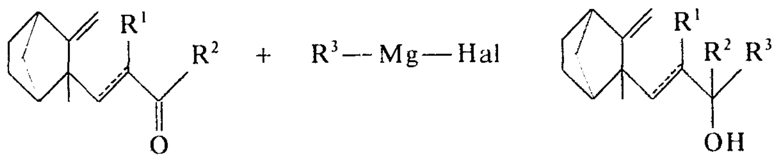

(Ia, Ib) (VI) (Ie bzw. If)

Der Duft der Alkohole, in denen $R^3$ für eine Methylgruppe steht, ist angenehm sandelholzartig.

Der Duft der Alkohole, in denen $R^3$ für eine Vinylgruppe steht, ist etwas schwächer holzig.

Ausgehend von Ie bzw. If, in denen $R^3$ für die Vinylgruppe steht, lassen sich auf an sich bekannte Weise die Verbindungen VII herstellen, unter denen sich VIIa und VIIb durch einen besonders feinen, herb-frischen Sandelholzgeruch auszeichnen.

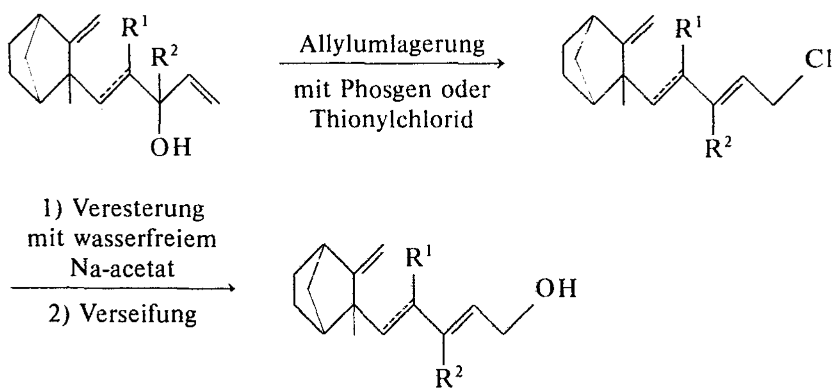


(VII)

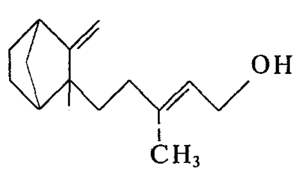

(VIIa)

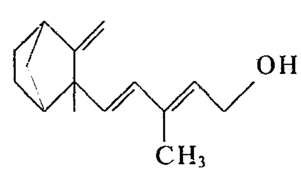

(VIIb)

Im Vergleich zum $\beta$-Santalol (Va) und zum Dehydro-$\beta$-Santalol (Vb) erscheint hier die Methylgruppe der Seitenkette um eine Position in Richtung des Ringes verschoben.

Die neuen bicyclischen Verbindungen besetzen teilweise wertvolle Duftstoffeigenschaften, die anderen eröffnen vorteilhafte Wege zur synthetischen Herstellung des begehrten Sandelholzduftstoffs

β-Santalol und anderen chemisch verwandten Verbindungen mit interessanten holzigen Duftnoten.

Die Struktur der gemäß den folgenden Beispielen hergestellten Verbindungen wurde mittels Elementaranalyse, IR- und NMR-Spektroskopie bestätigt.

Beispiel 1

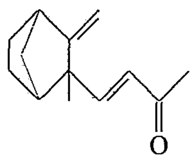

2-Methylen-3-methyl-3-(but-1-en-3-on-1-yl)-bicyclo-[2,2,1]-heptan

Zu einer Lösung aus 2 g KOH in 5 ml Methanol wurde im Laufe von 10 Minuten eine Lösung aus 50 g (0,33 Mol) 2-Methylen-3-methyl-3-formyl-bicyclo-[2,2,1]-heptan (II) in 80 g (1,38 Mol) Aceton gegeben, das Reaktionsgemisch wurde noch 3 Stunden unter Rückflußkühlung zum Sieden erhitzt und danach mit Eisessig neutralisiert. Nach Abtrennung des Kaliumacetates lieferte die übliche destillative Aufarbeitung das obengenannte Verfahrensprodukt in 59%iger Ausbeute.

Kp = 80—85°C bei 0,4 mbar.

$n_D^{25}$ = 1,5119.

Duftcharakteristik: schwach holzig.

Beispiel 2

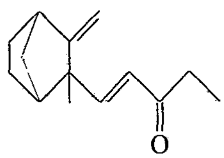

2-Methylen-3-methyl-3-(pent-1-en-3-on-1-yl)-bicyclo-[2,2,1]-heptan

Analog Beispiel 1 wurde eine Lösung von 20 g KOH in 60 ml Wasser mit einer Lösung aus 272 g (1,83 Mol) 2-Methylen-3-methyl-3-formyl-bicyclo-[2,2,1]-heptan und 810 g (11,2 Mol) Methyläthylketon zu der obengenannten Verbindung umgesetzt.

Ausbeute: 84%.

Kp = 85°C bei 0,1 mbar.

$n_D^{25}$ = 1,5089.

Duftcharakteristik: schwach holzig.

Beispiel 3

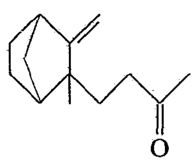

2-Methylen-3-methyl-3-(butan-3-on-1-yl)-bicyclo-[2,2,1]-heptan

27 g (0,142 Mol) des Ketones gemäß Beispiel 1 erhaltenen 2-Methylen-3-methyl-3-(but-1-en-3-on-1-yl)-bicyclo-[2,2,1]-heptans wurden mit 0,5 g Raney-Nickel, welches in 200 ml Methanol suspendiert war, bei 25°C und Normaldruck mit Wasserstoff hydriert. Nach Absättigung der olefinischen Doppelbindung in der Seitenkette wurde kein weiterer Wasserstoff aufgenommen. Die übliche destillative Aufarbeitung liefert das obengenannte Verfahrensprodukt in 93%iger Ausbeute.

Kp = 70°C bei 0,1 mbar.

$n_D^{25}$ = 1,4910.

Duftcharakteristik: frisch, holzig.

Beispiel 4

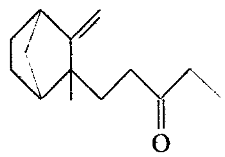

2-Methylen-3-methyl-3-pentan-3-on-1-yl)-bicyclo-[2,2,1]-heptan

20,4 g (0,1 Mol) des gemäß Beispiel 2 erhaltenen 2-Methylen-3-methyl-(pent-1-en-3-on-1-yl)-bicyclo-[2,2,1]-heptans wurden mit 0,25 g eines handelsüblichen Palladium-Calciumcarbonat-Kontaktes, der in 200 ml Methanol suspendiert war, bei 25°C und Normaldruck hydriert.

Die übliche Aufarbeitung lieferte die obengenannte Verbindung in 91%iger Ausbeute.

Kp = 87°C bei 0,2 mbar.

$n_D^{25}$ = 1,4912.

Duftcharakteristik: frisch, holzig.

Beispiel 5

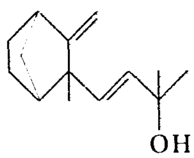

2-Methylen-3-methyl-3-(3-methyl-but-1-en-3-ol-1-yl)-bicyclo-[2,2,1]-heptan

Eine Lösung aus 0,12 Mol Methylmagnesiumchlorid in 75 ml Tetrahydrofuran wurde allmählich mit 19 g (0,1 Mol) des gemäß Beispiel 1 erhaltenen 2-Methylen-3-methyl-3-(but-1-en-3-on-1-yl)-bicyclo-[2,2,1]-heptans versetzt, wonach das Reaktionsgemisch noch 16 Stunden gerührt wurde.

Die übliche Aufarbeitung dieses Grignard-Ansatzes lieferte das obengenannte Carbinol in 73%iger Ausbeute.

Kp = 83°C bei 0,3 mbar.

$n_D^{25}$ = 1,5108.

Duftcharakteristik: sandelholzartig.

Beispiel 6

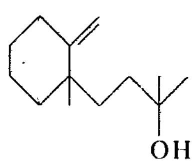

2-Methylen-3-methyl-3-(3-methyl-butan-3-ol-1-yl)-bicyclo-[2,2,1]-heptan

Diese Verbindung wurde analog Beispiel 5 durch Umsetzung des gemäß Beispiel 3 erhaltenen 2-Methylen-3-methyl-3-(butan-3-on-1-yl)-bicyclo-[2,2,1]-heptans mit Methylmagnesiumchloridlösung in 83%iger Ausbeute hergestellt.

Kp = 85°C bei 0,3 mbar.

$n_D^{25}$ = 1,4928.

Duftcharakteristik: angenehm holzig.

## Beispiel 7

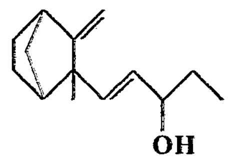

2-Methylen-3-methyl-3-(pent-1-en-3-ol-1-yl)-bicyclo-[2,2,1]-heptan

Eine Lösung aus 1,5 g (0,04 Mol) $LiAlH_4$ und 100 ml Tetrahydrofuran wurde bei Raumtemperatur langsam mit einer Lösung aus 20,4 g (0,1 Mol) des gemäß Beispiel 2 erhaltenen 2-Methylen-3-methyl-3-(pent-1-en-3-on-1-yl)-bicyclo-[2,2,1]-heptans in 20 ml Tetrahydrofuran versetzt, wonach das Gemisch 2 Stunden lang unter Rückfluß zum Sieden erhitzt wurde. Die übliche Aufarbeitung lieferte das obengenannte Verfahrensprodukt in 68%iger Ausbeute.
Kp = 92°C bei 0,4 mbar.
$n_D^{25}$ = 1,5018.
Duftcharakteristik: schwach holzig.

## Beispiel 8

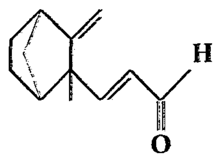

2-Methylen-3-methyl-3-(prop-1-en-3-al-1-yl)-bicyclo-[2,2,1]-heptan

Eine Lösung aus 0,4 Mol Vinylmagnesiumchlorid in 125 ml Tetrahydrofuran wurde allmählich mit einer Lösung aus 30 g (0,33 Mol) des Aldehyds II versetzt, das Reaktionsgemisch 16 Stunden lang gerührt und wie üblich aufgearbeitet. Hierbei fiel 2-Methylen-3-methyl-3(prop-1-ol-2-en-1-yl)-bicyclo-[2,2,1]-heptan in 73%iger Ausbeute an.
Kp = 60–63°C bei 0,3 mbar.
$n_D^{25}$ = 1,5089.
17,8 g (0,1 Mol) des erhaltenen Alkohols wurden zusammen mit 8 g Dimethylformamid, 0,2 g CuCl und 30 ml Toluol vorgelegt, das Reaktionsgemisch inneralb von 20 Minuten bei 0 bis 5°C allmählich mit 12,5 g (0,105 Mol) Thionylchlorid versetzt und 16 Stunden bei Raumtemperatur gerührt. Diese unter gleichzeitiger Chlorierung verlaufende Allylumlagerung lieferte nach der üblichen Aufarbeitung, jedoch ohne Reindarstellung durch Destillation, die Chlorverbindung

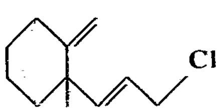

in einer Rohausbeute von 67%.
21,5 g (etwa 0,1 Mol) dieses rohen Chlorids wurden in 30 ml Äthanol gelöst und die Lösung innerhalb von 30 Minuten bei Raumtemperatur zu einer Lösung aus 8,8 g (0,13 Mol) Na-Äthylat, 11 g (0,124 Mol) 2-Nitro-propan und 100 ml Äthanol gegeben.
Das erhaltene Gemisch wurde sodann 3 Stunden lang bei 35°C und weitere 16 Stunden bei Raumtemperatur gerührt, nach Entfernung des Äthanols mit Wasser versetzt und mit verdünnter Schwefelsäure auf einen pH-Wert von 3 gebracht und anschließend mit Äther extrahiert.
Aus der Ätherphase wurde der obengenannte Aldehyd in 60%iger Ausbeute, bezogen auf eingesetztes rohes Chlorid gewonnen.
Kp = 65–70°C bei 0,1 mbar.
$n_D^{25}$ = 1,5190.
Duftcharakteristik: aldehydig, holzig.

Beispiel 9

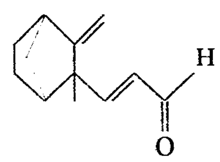

2-Methylen-3-methyl-3-(prop-1-en-3-al-1-yl)-bicyclo-[2,2,1]-heptan

22 g (etwa 0,1 Mol) der gemäß Beispiel 8 erhaltenen Chlorverbindung (2-Methylen-3-methyl-3-(3-chlor-prop-1-en-1-yl)-bicyclo-[2,2,1]-heptan) wurden zusammen mit einer Lösung aus 30 g Hexamethylentetramin in 200 g Chloroform 3 Stunden lang unter Rückflußkühlung erhitzt. Das hierbei anfallende Salz wurde abgetrennt und einer Wasserdampfdestillation unterworfen, wobei es sich in Methylamin und in 2-Methylen-3-methyl-3-(prop-1-en-3-al-1-yl)-bicyclo-[2,2,1]-heptan zersetzte. Die Ausbeute, bezogen auf das eingesetzte Chlorid betrug 21,6%.

Beispiel 10

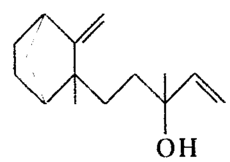

2-Methylen-3-methyl-3-(3-methyl-pent-4-en-3-ol-1-yl)-bicyclo-[2,2,1]-heptan

Diese Verbindung wurde durch Umsetzen mit Vinylmagnesiumchlorid analog der ersten Stufe von Beispiel 8 aus 22,7 g (0,118 Mol) eines gemäß Beispiel 3 erhaltenen 2-Methylen-3-methyl-3-(butan-3-on-1-yl)-bicyclo-[2,2,1]-heptans in 83%iger Ausbeute hergestellt.
Kp = 87°C bei 0,3 mbar.
$n_D^{25}$ = 1,5009.
Duftcharakteristik: schwach holzig.

Beispiel 11

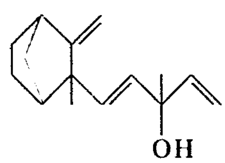

2-Methylen-3-methyl-3-(3-methyl-penta-1,4-dien-3-ol-1-yl)-bicyclo-[2,2,1]-heptan

Dieses Carbinol wurde durch Umsetzung von 37 g (0,24 Mol) eines gemäß Beispiel 1 erhaltenen 2-Methylen-3-methyl-(but-1-en-3-on-1-yl)-bicyclo-[2,2,1]-heptans mit Vinylmagnesiumchlorid analog der ersten Stufe von Beispiel 8 hergestellt. Die Ausbeute betrug 82%.
Kp = 78–80°C bei 0,1 mbar.
$n_D^{25}$ = 1,5062.
Duftcharakteristik: schwach holzig.

Beispiel 12

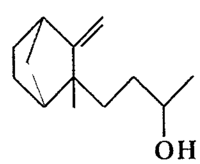

2-Methylen-3-methyl-3-(pentan-3-ol-1-yl)-bicyclo-[2,2,1]-heptan

Die Hydrierung des gemäß Beispiel 4 erhaltenen 2-Methylen-3-methyl-3-(pentan-3-on-1-yl)-bicyclo-[2,2,1]-heptans analog Beispiel 18 mit $NaBH_4$ lieferte die obengenannte Verbindung in 64%iger

Ausbeute.
Kp = 95°C bei 0,1 mbar.
$n_D^{25}$ = 1,4942.
Duftcharakteristik: herb, holzig.

Beispiel 13

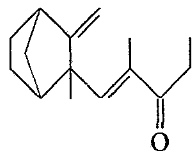

2-Methylen-3-methyl-3-(2-methyl-pent-1-en-3-on-1-yl)-bicyclo-[2,2,1]-heptan

30 g (0,2 Mol) des Aldehyds II und 120 g (1,4 Mol) Diäthylketon wurden analog Beispiel 1 miteinander umgesetzt. Die Reinausbeute an dem obengenannten Verfahrensprodukt betrug 18%.
Kp = 88–92°C bei 0,1 mbar.
Duftcharakteristik: frisch, holzig.

Beispiel 14

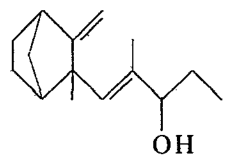

2-Methylen-3-methyl-3-(2-methyl-pent-1-en-3-ol-1-yl)-bicyclo-[2,2,1]-heptan

Durch Hydrierung des gemäß Beispiel 1 erhaltenen 2-Methylen-3-methyl-3-(but-1-en-3-on-1-yl)-bicyclo-[2,2,1]-heptans analog Beispiel 7 wurden die obengenannte Verbindung in 75%iger Ausbeute erhalten.
Kp = 130°C bei 0,07 mbar.
$n_D^{25}$ = 1,5074.
Duftcharakteristik: Sandelholz westindisch.

Beispiele für die Verwendung der erfindungsgemäßen Verbindungen für die Herstellung von β-Santalol, Dehydro-β-Santalol und anderen chemisch verwandten Verbindungen

Beispiel 15

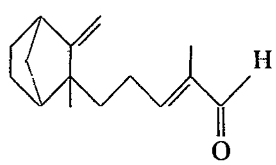

2-Methylen-3-methyl-3-(4-methyl-pent-3-en-5-al-1-yl)-bicyclo-[2,2,1]-heptan

Eine Lösung aus 40 g (0,77 Mol) Natriummethylat und 420 g (7 Mol) Methylformiat wurden bei Siedetemperatur im Laufe von einer Stunde mit 150 g (0,735 Mol) des gemäß Beispiel 2 erhaltenen 2-Methylen-3-methyl-3-(pent-1-en-3-on-1-yl)-bicyclo-[2,2,1]-heptans versetzt, wonach das Reaktionsgemisch noch weitere 3 Stunden unter Rückfluß zum Sieden erhitzt wurde. Diese Mischung wurde bei 15 bis 20°C mit einer Lösung aus 30 g (0,82 Mol) HCl in 120 ml Methanol versetzt, 16 Stunden lang gerührt und danach mit Natriummethylat neutralisiert.
Die Aufarbeitung des Reaktionsgemisches lieferte neben 75 g unumgesetzten Ausgangsmaterials 63 g des Acetals, entsprechend einer Ausbeute von 62%, bezogen auf umgesetztes Ausgangsmaterial vom Siedepunkt Kp = 170°C bei 0,2 mbar.

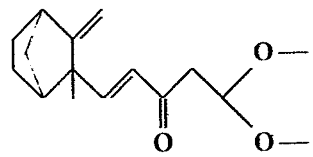

23,5 g (0,085 Mol) dieses Acetals wurden analog Beispiel 3 unter Absättigung der olefinischen Doppelbindung in der Seitenkette hydriert. Die Ausbeute an 2-Methylen-3-methyl-3-(4-methyl-5,5-dimethoxy-3-oxo-pent-1-en-1-yl)-bicyclo-[2,2,1]-heptan betrug 90%.
Kp = 130°C bei 0,04 mbar.
$n_D^{25}$ = 1,4928.
19 g (0,056 Mol) des hydrierten Acetals wurden analog Beispiel 7 mit $LiAlH_4$ in den Alkohol überführt.

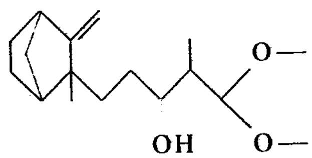

Kp = 140°C bei 0,1 mbar.
Ausbeute = 60%, bezogen auf eingesetztes rohes Keto-Acetal.
42 g (0,149 Mol) des erhaltenen Alkohols wurden zusammen mit 150 ml Eisessig, 25 ml Wasser und 20 g Natriumacetat 3 Stunden lang bei 90°C gerührt. Anschließend wurde das Reaktionsgemisch mit Wasser verdünnt und mit Petroläther extrahiert. Die Aufarbeitung der Petrolätherphase lieferte das Verfahrensprodukt, welches mit dem des Beispiels 20 identisch war, in 85%iger Ausbeute, bezogen auf das umgesetzte Acetal.

## Beispiel 16

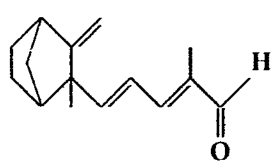

2-Methylen-3-methyl-3-(4-methyl-penta-1,3-dien-5-al-1-yl)-bicyclo-[2,2,1]-heptan

Diese Verbindung wurde analog Beispiel 15, jedoch ohne die Hydrierung der olefinischen Doppelbindung in der Seitenkette hergestellt. Die Ausbeute betrug 68%, bezogen auf die umgesetzte Ausgangsverbindung.
Kp = 86°C bei 0,07 mbar.
$n_D^{25}$ = 1,5305.
Duftcharakteristik: aldehydig, holzig.
Das hierbei als Zwischenstufe aufgetretene Alkenol

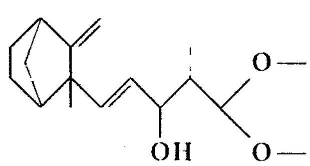

fiel in 80%iger Ausbeute an.
Kp = 160°C bei 0,3 mbar.

Beispiel 17

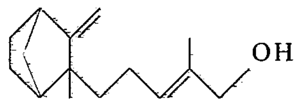

β-Santalol

Die Hydrierung des gemäß dem Beispiel 15 erhaltenen 2-Methylen-3-methyl-3(4-methyl-5-al-3-en-1-yl)-bicyclo-[2,2,1]-heptans analog Beispiel 7 lieferte das β-Santalol in 60%iger Ausbeute.
Kp = 101 bis 103°C bei 0,1 mbar.
Duftcharakteristik: Sandelholz ostindisch.

Beispiel 18

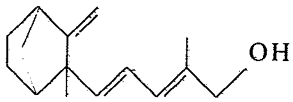

Dehydro-β-Santalol

Die Hydrierung des gemäß Beispiel 16 erhaltenen 2-Methylen-3-methyl-3-(4-methyl-penta-1,3-dien-5-al-1-yl)-bicyclo-[2,2,1]-heptans analog Beispiel 7, jedoch mit $NaBH_4$ anstelle des $NaAlH_4$, lieferte das Dehydro-β-Santalol in 69%iger Ausbeute.
Kp = 120 bis 122°C bei 0,3 mbar.
$n_D^{25}$ = 1,5411.
Duftcharakteristik: feiner Edelholzgeruch.

Beispiel 19

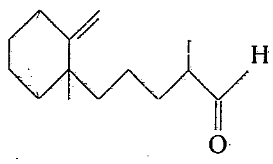

2-Methylen-3-methyl-3-(4-methyl-pentan-5-al-1-yl)-bicyclo-[2,2,1]-heptan

Durch Hydrierung eines gemäß Beispiel 15 erhaltenen 2-Methylen-3-methyl-3-(4-methyl-pent-3-en-5-al-1-yl)-bicyclo-[2,2,1]-heptans analog Beispiel 3 erhielt man den obengenannten gesättigten Aldehyd in praktisch quantitativer Ausbeute.
Kp = 97–100°C bei 0,3 mbar.
$n_D^{25}$ = 1,5029.
Duftcharakteristik: aldehydartig, holzig.

Beispiel 20

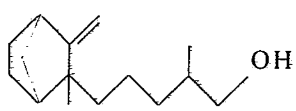

2-Methylen-3-methyl-3-(4-methyl-pentan-5-ol-1-yl)-bicyclo-[2,2,1]-heptan

Durch Hydrierung des gemäß Beispiel 19 erhaltenen Aldehyds analog Beispiel 7 erhielt man das obengenannte Verfahrensprodukt in 46%iger Ausbeute.
Kp = 140°C bei 0,4 mbar.
$n_D^{25}$ = 1,5136.
Duftcharakteristik: Sandelholz.

Beispiel 21

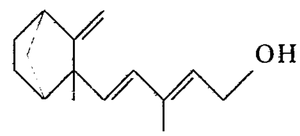

2-Methylen-3-methyl-3-(3-methyl-penta-1,3-dien-5-ol-1-yl)-bicyclo-[2,2,1]-heptan

Eine Lösung aus 32 g (0,147 Mol) eines gemäß Beispiel 11 erhaltenen 2-Methylen-3-methyl-3-(3-methyl-penta-1,4-dien-3-ol-1-yl)-bicyclo-[2,2,1]-heptans, 15 ml Dimethylformamid und 30 ml Toluol wurde bei 0°C langsam mit 18 g (0,151 Mol) Thionylchlorid versetzt und danach noch 3 Stunden lang bei Raumtemperatur gerührt. Die übliche Aufarbeitung lieferte 36,6 g des rohen, aus Allylumlagerung und gleichzeitiger Chlorierung des Einsatztoffes hervorgegangenen Chlorids

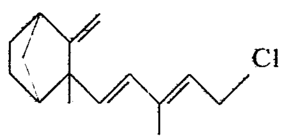

Das erhaltene rohe Chlorid wurde anschließend in einer Suspension aus 30 g wasserfreiem Natriumacetat, 0,5 g Natriumjodid, 1 ml Triäthylamin, 15 ml Dimethylformamid und 30 ml Toluol im Laufe von 8 Stunden bei 20°C in das Acetat überführt. Das Acetat wude in methanolisch-wäßriger Natronlauge wie üblich verseift. Die Aufarbeitung des Reaktionsgemisches lieferte das obengenannte Dienol.
Kp = 111°C bei 0,07 mbar.
$n_D^{25}$ = 1,5411.
Geruchscharakteristik: holzig.

Beispiel 22

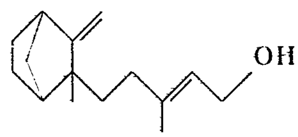

2-Methylen-3-methyl-3-(3-methyl-pent-3-en-5-ol-1-yl)-bicyclo-[2,2,1]-heptan

Diese Verbindung wurde analog Beispiel 21 aus 74 g (0,337 Mol) des gemäß Beispiel 10 erhaltenen 2-Methylen-3-methyl-3-(3-methyl-pent-4-en-3-ol-1-yl)-bicyclo-[2,2,1]-heptans in 90%iger Ausbeute hergestellt, wobei jedoch anstelle des Thionylchlorids Phosgen als Halogenierungs- und Umlagerungsreagens verwendet wurde.
Kp = 110–115°C bei 0,3 mbar.
$n_D^{25}$ = 1,5125.
Duftcharakteristik: intensiver, feiner Edelholzgeruch.

**Patentansprüche**

1. Bicyclische Verbindungen der allgemeinen Formel I

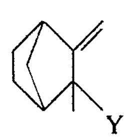

(I)

in der Y für

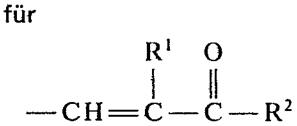

(Ia)

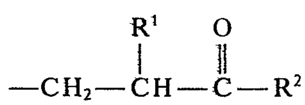

(Ib)

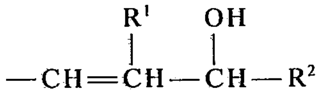

(Ic)

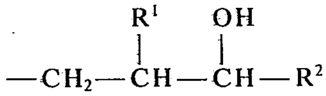

(Id)

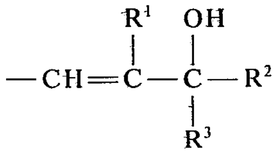

(Ie)

oder

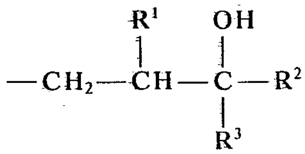

(If)

steht,
worin

$R^1$ Wasserstoff oder die Methylgruppe,
$R^2$ Wasserstoff, eine Methyl- oder Äthylgruppe und
$R^3$ eine Methyl- oder Vinylgruppe bedeuten, ausgenommen die Verbindung der allgemeinen Formel Ib, in der $R^1 = R^2$ = Wasserstoff sind.

2. Verfahren zur Herstellung der bicyclischen Verbindungen der allgemeinen Formel I gemäß Anspruch 1, dadurch gekennzeichnet, daß man den Aldehyd II

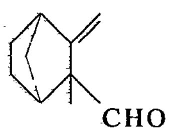

(II)

in an sich bekannter Weise nach den Methoden der gemischten Aldolkondensation mit einer Carbonylverbindung der allgemeinen Formel III

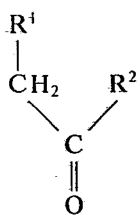

(III)

in der $R^1$ und $R^2$ die in Anspruch 1 angegebene Bedeutung haben, umsetzt und die hierbei entstehende Verbindung der Formel Ia gewünschtenfalls in an sich bekannter Weise zu den Verbindungen der Formeln Ib, Ic bzw. Id hydriert oder aber zur Herstellung der Verbindungen Ie und If die entstehenden Verbindungen Ia bzw. Ib in an sich bekannter Weise unter den Bedingungen von Grignard-Reaktionen mit einer Grignardverbindung der allgemeinen Formel VI

$$R^3\text{—Mg—Hal} \qquad \text{(VI)}$$

in der $R^3$ eine Methyl- oder Vinylgruppe bedeutet und Hal für Chlor oder Brom steht, umsetzt.

3. Verfahren zur Herstellung der bicyclischen Verbindung der allgemeinen Formel Ia

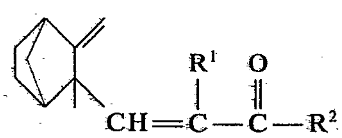

(Ia)

in der $R^1 = R^2 = H$ sind, dadurch gekennzeichnet, daß man A den Aldehyd II

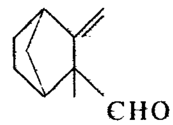

(II)

in an sich bekannter Weise mit Vinyl-Magnesiumhalogeniden umsetzt, B den erhaltenen Allylalkohol (2-Methylen-3-methyl-3-(1-hydroxy-2-propen-1-yl)-bicyclo[2,2,1]-heptan)

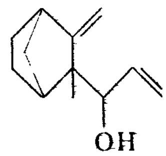

in an sich bekannter Weise mit Phosgen oder Thionylchlorid in das Chlorid

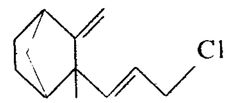

umlagert und C das erhaltene Chlorid in an sich bekannter Weise oxydiert.

4. Verwendung der bicyclischen Verbindungen der allgemeinen Formeln Ia oder Ib gemäß Anspruch 1 zur Herstellung von Verbindungen der allgemeinen Formel V

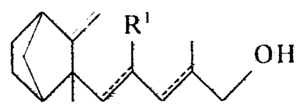

(V)

in der $R^1$ für Wasserstoff oder eine Methylgruppe steht und die Bindungen ---- eine Doppelbindung oder eine aus der Doppelbindung durch Hydrierung hervorgegangene Einfachbindung bedeuten, durch Umsetzen der Verbindungen der Formeln Ia bzw. Ib in an sich bekannter Weise nach den Methoden der gemischten Aldolkondensation mit Propionaldehyd und anschließendes Hydrieren in an sich bekannter Weise.

5. Verwendung der bicyclischen Verbindungen der allgemeinen Formeln Ia oder Ib, in denen $R^1$ für H und $R^2$ für Äthyl steht, zur Herstellung von β-Santalol Va bzw. Dehydro-β-Santalol Vb

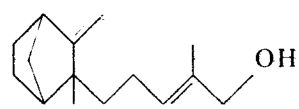

(Va)

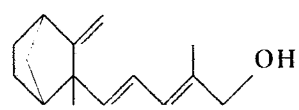

(Vb)

durch Umsetzen der Ausgangsverbindungen mit Methylformiat in Gegenwart von niederen Alkalialkoholaten, Acetalisieren mit Methanol in Gegenwart saurer Katalysatoren, Hydrieren der Carbonylgruppe, Dehydratisieren, Desacetalisieren und Hydrieren der freigesetzten Formylgruppe in an sich bekannter Weise.

**Claims**

1. Bicyclic compounds of the general formula I

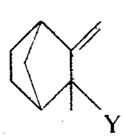

(I)

where Y is

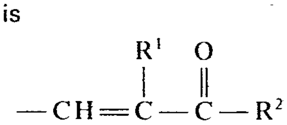

(Ia)

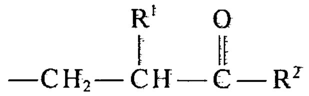

(Ib)

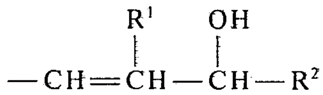

(Ic)

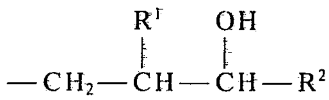

(Id)

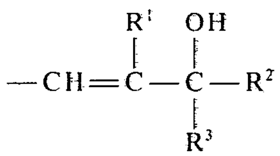

(Ie)

or

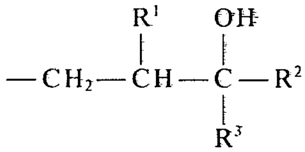

(If)

where $R^1$ is hydrogen or methyl, $R^2$ is hydrogen, methyl or ethyl and $R^3$ ist methyl or vinyl, with the exception of the compound of the general formula Ib where $R^1$ and $R^2$ are both hydrogen.

2. A process for the production of the bicyclic compounds of the general formula I as claimed in claim 1, characterized in that the aldehyde II

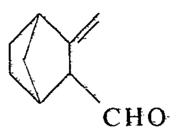

(II)

is reacted in the conventional manner, by the mixed aldol condensation methods, with a carbonyl compound of the general formula III

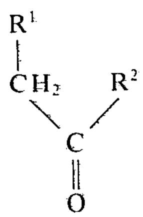

(III)

where $R^1$ and $R^2$ have the meanings given in claim 1, and, if desired, the resulting compound of the formula Ia is hydrogenated in the conventional manner to give a compound of the formula Ib, Ic or Id or, in order to prepare a compound Ie or If, the compound Ia or Ib formed is reacted in the conventional manner, under the Grignard reaction conditions, with a Grignard compound of the general formula VI

$$R^3—Mg—Hal \qquad (VI)$$

where $R^3$ is methyl or vinyl and Hal is chlorine or bromine.

3. A process for the production of the bicyclic compounds of the general formula Ia

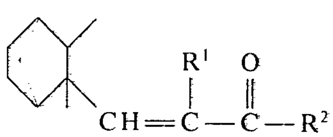

(Ia)

where $R^1$ and $R^2$ are both hydrogen, characterized in that (A) the aldehyde II

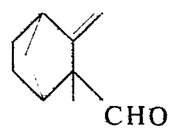

(II)

is reacted in the conventional manner with vinyl-Mg halide, (B) the resulting allyl alcohol (2-methylene-3-methyl-3-(1-hydroxy-2-propen-1-yl)-bicyclo[2,2,1]-heptane)

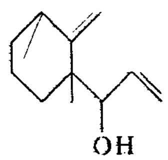

is rearranged in the conventional manner with phosgene or thionyl chloride to give the chloride

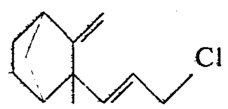

and (C) the resulting chloride is oxidized in the conventional manner.

4. Use of a bicyclic compound of the general formula Ia or Ib as claimed in claim 1 for the preparation of a compound of the general formula V

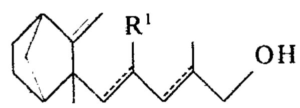

(V)

where $R^1$ ist hydrogen or methyl and the bond ---- is a double bond or a single bond produced from the double bond by hydrogenation, by reacting a compound of the formula Ia or Ib with propionaldehyde in the conventional manner, by the mixed aldol condensation methods, and then hydrogenating the product in the conventional manner.

5. Use of a bicyclic compound of the general formula Ia or Ib, where $R^1$ ist H and $R^2$ ist ethyl, to prepare $\beta$-santalol Va or dehydro-$\beta$-santalol Vb

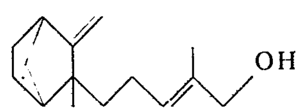

(Va)

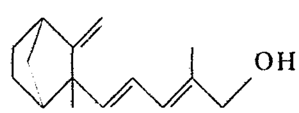

(Vb)

by reacting the starting compound with methyl formate in the presence of a lower alcoholate of an alkali metal, acetalizing the product with methanol in the presence of an acid catalyst, hydrogenating the carbonyl group, dehydrating and desacetalizing the product and hydrogenating the liberated formyl group, the above reactions being carried out in the conventional manner.

## Revendications

1. Composés bicycliques de formule générale I

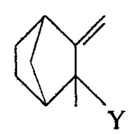

(I)

dans laquelle Y est mis pour

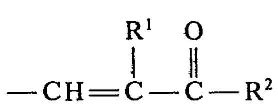

(Ia)

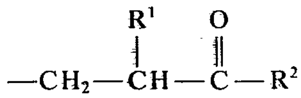

(Ib)

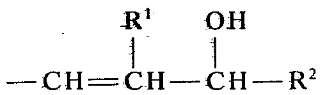

(Ic)

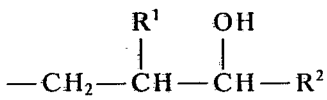

(Id)

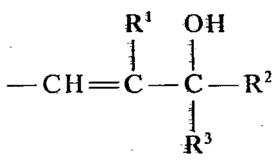

(Ie)

ou

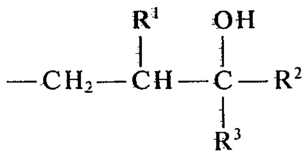

(If)

où

$R^1$ est un hydrogène ou le groupe méthyle,
$R^2$ est un hydrogène ou un groupe méthyle ou éthyle et
$R^3$ est un groupe méthyle ou vinyle, excepté le composé de formule générale Ib dans laquelle $R^1 = R^2 =$ hydrogène.

2. Procédé pour la préparation des composés bicycliques de formule générale I selon la revendication 1, caractérisé en ce qu'on fait réagir l'aldéhyde II

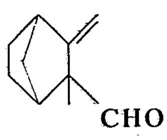

(II)

de façon connue en soi, suivant les méthodes de l'aldolisation mixte, avec un composé carbonylé de formule générale III

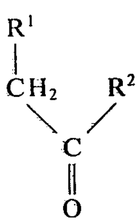

(III)

dans laquelle $R^1$ et $R^2$ ont les significations données dans la revendication 1 et, si on le désire, on transforme par hydrogénation le composé Ia ainsi obtenu, de façon connue en soi, en les composés de formules Ib, Ic ou Id ou, pour la préparation des composés Ie et If, on fait réagir le composé Ia ou Ib formé, de façon connue en soi, dans les conditions de réactions de Grignard, aves un composé de Grignard de formule générale VI

$$R^3—Mg—Hal \qquad (VI)$$

dans laquelle $R^3$ représente un groupe méthyle ou vinyle et Hal est mis pour un chlore ou un brome.

3. Procédé pour la préparation du composé bicyclique de formule générale Ia

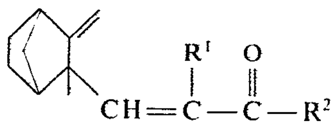

(Ia)

dans laquelle $R^1 = R^2 = H$, caractérisé en ce qu'on fait réagir l'aldéhyde II

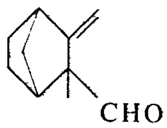

(II)

de façon connue en soi avec des halogénures de vinyl-magnésium, on transpose l'alcool allylique 2-méthylène-3-méthyl-3-(1-hydroxy-2-propène-1-yl)-bicyclo-[2,2,1]-heptane obtenu

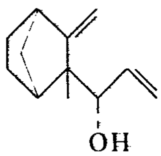

de façon connue en soi, avec du phosgène ou du chlorure de thionyle, en le chlorure

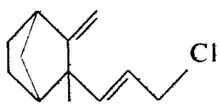

et on oxyde de façon connue en soi le chlorure obtenu.

4. Utilisation des composés bicycliques de formule générale Ia ou Ib selon la revendication 1 pour la préparation de composés de formule V

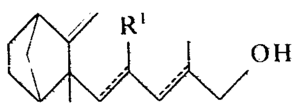

(V)

dans laquelle $R^1$ est mis pour un hydrogène ou un groupe méthyle et les liaisons ---- représentent une double liaison ou une liaison simple provenant de la double liaison par hydrogénation, par réaction des composés de formule Ia ou Ib de façon connue en soi, suivant les méthodes de l'aldolisation mixte, avec l'aldéhyde propionique, suivie d'une hydrogénation de façon connue en soi.

5. Utilisation des composés bicycliques de formule générale Ia ou Ib, dans laquelle $R^1$ est mis pour H et $R^2$ pour un éthyle, pour la préparation de $\beta$-santalol Va ou de déshydro-$\beta$-santalol Vb

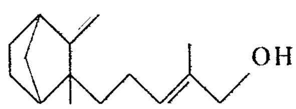

(Va)

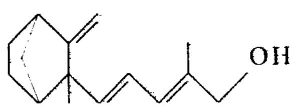

(Vb)

par réaction des composés de départ avec le formiate de méthyle en présence d'alcoolates alcalins inférieurs, acétalisation avec le méthanol en présence de catalyseurs acides, hydrogénation du groupe carbonyle, déshydratation, désacétalisation et hydrogénation du groupe formyle libéré de façon connue en soi.